# EUROPEAN PATENT APPLICATION

(11) **EP 4 553 160 A1**
(43) Date of publication of application: **14.05.2025**
(21) Application number: 23835567.1
(22) Date of filing: 05.07.2023
(51) Int. Cl.: C12N 15/61, A61K 38/52, A61K 48/00, A61P 31/12, C07K 16/40, C12N 9/90, C12Q 1/533

(54) **ANTIVIRAL AGENT, PHARMACEUTICAL COMPOSITION FOR TREATING OR PREVENTING VIRAL INFECTION, KIT FOR EVALUATING RISK OF VIRAL INFECTION BECOMING SEVERE, AND METHOD FOR EVALUATING RISK OF VIRAL INFECTION BECOMING SEVERE**

(30) Priority: 05.07.2022 JP 2022108705
(71) Applicant: Tohoku University, Sendai-shi, Miyagi 980-8577 (JP)
(72) Inventor: OKUMURA Masaki, Sendai-shi, Miyagi 980-8577 (JP); TAKAYAMA Kazuo, Kyoto-shi, Kyoto 606-8501 (JP); HASHIMOTO Rina, Kyoto-shi, Kyoto 606-8501 (JP); KANEMURA Shingo, Sendai-shi, Miyagi 980-8577 (JP)
(74) Representative: Plasseraud IP
(86) International application number: PCT/JP2023/024953
(87) International publication number: WO 2024/010036

(57) **Abstract**

An antiviral agent contains a protein having a disulfide bond cleavage activity as an active ingredient. A pharmaceutical composition for treating or preventing a virus infection contains the viral agent and a pharmaceutically acceptable carrier. A virus infection severity risk evaluation kit contains a specific binding substance for a protein having a disulfide bond cleavage activity. A virus infection severity risk evaluation method includes a step of measuring a blood concentration of a protein having a disulfide bond cleavage activity in a biological sample from a test subject.

## Description

### Technical Field

The present invention relates to an antiviral agent, a pharmaceutical composition for treating or preventing a virus infection, a virus infection severity risk evaluation kit, and a virus infection severity risk evaluation method.

The present application claims priority to Japanese Patent Application No. 2022-108705 filed in Japan on July 5, 2022, the contents of which are incorporated herein.

### Background Art

As seen from the global epidemic of COVID-19, rapid development of therapeutic drugs for emerging virus infections has become a global challenge. The therapeutic drugs for virus infections are often developed by targeting a protein possessed by a causative virus.

For example, an antibody drug that inhibits a binding between a spike receptor-binding domain (RBD) of SARS-CoV-2 and human angiotensin I-converting enzyme 2 (hACE2), an antagonist, and the like are being developed as a therapeutic drug for COVID-19. However, if mutations occur in an RBD, an ability to recognize the RBD may be reduced, and an infection inhibiting effect may be reduced in antibodies and antagonists.

A protein disulfide isomerase (PDI) family is a group of proteins that have a thioredoxin-like domain localized in endoplasmic reticulum in cells. Approximately 20 kinds of PDI family proteins are known in mammals. It is reported that protein disulfide isomerase (PDI), which is one of the PDI family proteins, has functions such as a thiol-disulfide oxidoreductase activity, a disulfide isomerase activity, and a redox-dependent chaperone activity (NPL 1). It is also shown that the PDI family exists extracellularly in the brain and blood, and it is reported that cholera toxin is unfolded by a disulfide reductase activity as an extracellular function (NPL 2).

### Citation List

### Non Patent Literature

NPL 1: Hyder Ali Khan and Bulent Mutus, Protein disulfide isomerase a multifunctional protein with multiple physiological roles. Front Chem. 2014 Aug 26; 2:70.
NPL 2: B Tsai et al., Protein disulfide isomerase acts as a redox-dependent chaperone to unfold cholera toxin. Cell. 2001 Mar 23;104(6):937-48.

### Summary of Invention

### Technical Problem

A drug targeting a virus protein possessed by a causative virus have to be developed for each causative virus, and thus it is impossible to respond quickly to an emerging virus infection. An antiviral activity against mutant strains in which a mutation occurs in a target protein may be reduced. A drug that inhibits a binding between a host cell receptor and a causative virus cannot neutralize the causative virus remaining in the body.

Therefore, an object of the invention is to provide an antiviral agent that can maintain an antiviral activity against a mutant strain of a causative virus of a virus infection, and a pharmaceutical composition for treating or preventing a virus infection and containing the antiviral agent. In addition, an object of the invention is to provide a virus infection severity risk evaluation kit, and a virus infection severity risk evaluation method.

### Solution to Problem

The invention includes the following aspects.
[1] An antiviral agent containing:
   at least one selected from the group consisting of (a) a protein having a disulfide bond cleavage activity, and (b) a polynucleotide containing a nucleotide sequence encoding the protein.
[2] The antiviral agent according to [1], in which the protein is a protein disulfide isomerase family protein.
[3] The antiviral agent according to [2], in which the protein disulfide isomerase family protein is at least one selected from the group consisting of PDI, **ERp46,** ERp57, ERp72, and P5.
[4] The antiviral agent according to [3], in which the protein disulfide isomerase family protein is at least one selected from the group consisting of PDI and ERp46.
[5] The antiviral agent according to any one of [1] to [4], which is to be applied to a virus having a disulfide bond in at least one protein selected from the group consisting of a spike protein, an envelope protein, and a capsid protein.
[6] A pharmaceutical composition for treating or preventing a virus infection, the pharmaceutical composition containing:
   the antiviral agent according to any one of [1] to [5]; and
   a pharmaceutically acceptable carrier.
[7] A virus infection severity risk evaluation kit containing:
   a specific binding substance for a protein having a disulfide bond cleavage activity.
[8] A virus infection severity risk evaluation method including:
   a step of measuring a concentration of a protein having a disulfide bond cleavage activity in a biological sample from a test subject.
[9] A determination method including:
   a step of measuring a concentration of a protein having a disulfide bond cleavage activity in a biological sample from a test subject;
   a step of evaluating a severity risk of a virus infection in the test subject based on the concentration of the protein having the disulfide bond cleavage activity; and
   a step of determining whether the test subject determined to have a high severity risk is a test subject to be administered with an antiviral agent.

### Advantageous Effects of Invention

According to the invention, an antiviral agent that can maintain an antiviral activity against a mutant strain of a causative virus of a virus infection, and a pharmaceutical composition for treating or preventing a virus infection and containing the antiviral agent are provided. In addition, a virus infection severity risk evaluation kit and a virus infection severity risk evaluation method are provided.

### Brief Description of Drawings

[FIG. 1] FIG. 1 shows a three-dimensional structure of PDI. Active sites for an antiviral activity are shown in the figure as redox-active sites.
[FIG. 2] FIG. 2 shows domain structures of PDI family proteins. "CGHC" indicates a CGHC motif in the domain.
[FIG. 3] FIG. 3 shows results of evaluation of a disulfide bond cleavage activity of each of the PDI family proteins by using an RBD of SARS-CoV-2.
[FIG. 4] FIG. 4 shows results of evaluation of an antiviral activity of each of the PDI family proteins against SARS-CoV-2.
[FIG. 5] FIG. 5 shows results of evaluation of cytotoxicity of PDI against human cells.
[FIG. 6] FIG. 6 shows results of evaluation of the disulfide bond cleavage activity of PDI by using an RBD of a SARS-CoV-2 Delta strain.
[FIG. 7] FIG. 7 shows results of evaluation of the disulfide bond cleavage activity of PDI by using an RBD of a SARS-CoV-2 Omicron strain.
[FIG. 8] FIG. 8 shows results of evaluation of an antiviral activity of PDI against the SARS-CoV-2 Delta strain.
[FIG. 9] FIG. 9 shows results of evaluation of an antiviral activity of PDI against the SARS-CoV-2 Omicron strain.
[FIG. 10] FIG. 10 shows results of evaluation of the disulfide bond cleavage activity of each of the PDI family proteins by using an RBD of hCoV-229E.

### Description of Embodiments

### [Definition]

A term "contain" (comprise) means that components other than the target component may be included. A term "consist of" means that components other than the target component are not included. A term "consist essentially of" means that components other than the target component in an aspect that exerts a special function (such as an aspect that completely loses an effect of the invention) are not included. In the present specification, when "contain" (comprise) is described, aspects of "consist of" and "consist essentially of" are included.

A numerical range expressed with "to" means a numerical range that includes numerical values written before and after **"to"** as lower and upper limit values.

A protein, a peptide, a polynucleotide (DNA, RNA), a vector, and a cell may be isolated. The term "isolated" means a natural state or a state of being separated from other components. An "isolated" one may substantially not contain other components. The expression of "substantially not contain other components" means that a content of other components contained in an isolated component is negligible. The content of other components contained in the isolated component may be, for example, 10% by mass or less, 5% by mass or less, 4% by mass or less, 3% by mass or less, 2% by mass or less, 1% by mass or less, 0.5% by mass or less, or 0.1% by mass or less. The protein, the peptide, the polynucleotide (DNA, RNA), the vector, and the cell described in the present specification may be an isolated protein, an isolated peptide, an isolated polynucleotide (isolated DNA, isolated RNA), an isolated vector, and an isolated cell, respectively.

The "polynucleotide" refers to a nucleotide polymer in which nucleotides are linked by a phosphodiester bond. The "polynucleotide" and a "nucleic acid" may be DNA, RNA, or a combination of DNA and RNA. The "polynucleotide" may be a polymer of natural nucleotides, a polymer of natural nucleotides and non-natural nucleotides (analogs of natural nucleotides, nucleotides in which at least one of a base moiety, a sugar moiety, or a phosphate moiety is modified (for example, phosphorothioate backbone), and the like), or a polymer of non-natural nucleotides.

A nucleotide sequence of the "polynucleotide" is written in the generally accepted single-letter code unless otherwise specified. The nucleotide sequence is written from a 5' side to a 3' side unless otherwise specified. Nucleotide residues constituting the "polynucleotide" may be written simply as adenine, thymine, cytosine, guanine, uracil, or the like, or in a single-letter code thereof.

The "polypeptide" and the "protein" are used interchangeably and refer to a polymer of amino acids linked by amide bonds. The "polypeptide" or the "protein" may be a polymer of natural amino acids, a polymer of natural amino acids and non-natural amino acids (chemical analogs, modified derivatives, or the like of natural amino acids), or a polymer of non-natural amino acids.

An amino acid sequence of the "polypeptide" or the "protein" is written in a generally accepted single-letter or three-letter code unless otherwise indicated. The amino acid sequence is written from an N-terminus to a C-terminus unless otherwise indicated.

A sequence identity (or homology) between nucleotide sequences or amino acid sequences is obtained by aligning two nucleotide sequences or amino acid sequences with gaps at insertion and deletion moieties such that corresponding bases or amino acids are most frequently matched, and calculating a ratio of the matched bases or amino acids to the entire nucleotide sequences or the entire amino acid sequences excluding the gaps in the obtained alignment. The sequence identity between the nucleotide sequences or the amino acid sequences can be determined using various kinds of homology search software known in the related art. For example, a value of the sequence identity of the nucleotide sequences can be obtained by calculation based on an alignment obtained by well-known homology search software BLASTN, and a value of the sequence identity of the amino acid sequences can be obtained by calculation based on an alignment obtained by well-known homology search software

### BLASTP.

A term "codon optimization" refers to replacing at least one codon in an original nucleotide sequence with a codon that is more frequently used in a target organism while maintaining an original amino acid sequence. A codon usage table is readily available, for example, at the "Codon Usage Database" (www.kazusa.or.jp/codon/) provided by the Kazusa DNA Research Institute Foundation. For example, codons can be optimized using the codon usage table. Computer algorithms for codon-optimizing a particular sequence for expression in particular animal species are also well-known. The computer algorithms for the codon optimization are available, for example, from Gene Forge (Aptagen, LLC.; Jacobus, PA).

Regarding the polynucleotide, a term "functionally link" means that a first nucleotide sequence is located sufficiently close to a second nucleotide sequence, and the first nucleotide sequence can affect the second nucleotide sequence or a region under the control of the second nucleotide sequence. For example, a description that the polynucleotide is functionally linked to a promoter means that the polynucleotide is linked to be expressed under the control of the promoter.

A "promoter is functional" means that the promoter can express the polynucleotide functionally linked to the promoter in cells of a target organism.

A term "expressible state" refers to a state where the polynucleotide can be transcribed in a cell into which the polynucleotide is introduced.

A term "expression vector" is a vector containing a target polynucleotide, and refers to a vector including a system that makes the target polynucleotide in the expressible state in a cell into which the vector is introduced.

A "virus infection" means a disease caused by infection with a virus. For example, an infection with SARS-CoV-2 causes the novel coronavirus infection (COVID-19).

A "causative virus" means a virus that causes the virus infection. For example, a causative virus of COVID-19 is SARS-CoV-2.

A "severity risk of a virus infection" means a risk that symptoms of the virus infection progress and become severe.

### [Antiviral Agent]

A first aspect of the invention is an antiviral agent. In one embodiment, the antiviral agent contains at least one selected from the group consisting of (a) a protein having a disulfide bond cleavage activity, and (b) a polynucleotide containing a nucleotide sequence encoding the protein.

### <Protein Having Disulfide Bond Cleavage Activity (Disulfide Bond Cleavage Enzyme)>

In one embodiment, the antiviral agent may contain the protein having the disulfide bond cleavage activity (hereinafter, also referred to as a "disulfide bond cleavage enzyme").

The "disulfide bond cleavage activity" refers to an activity of catalyzing a cleavage reaction of a disulfide bond. In one embodiment, the disulfide bond is a disulfide bond formed between thiol groups of two cysteine residues in a protein. The disulfide bond is involved in protein folding. When the disulfide bond is formed between the cysteine residues contained in the protein, the protein is folded and stabilized to become a protein in a native state. On the other hand, when the disulfide bond is cleaved, the protein is unfolded and destabilized. When the protein is unfolded, a function of the protein in the native state is lost.

Whether a target protein has the disulfide bond cleavage activity can be confirmed by a well-known method. Examples of the method for confirming the presence or absence of the disulfide bond cleavage activity include methods described in Examples. Specifically, the target protein is reacted with a substrate protein (protein containing the disulfide bond) in the presence of an appropriate reducing agent (for example, reduced glutathione). Then, cleavage of the disulfide bond in the substrate protein is confirmed. As a result, if the cleavage of the disulfide bond in the substrate protein is confirmed, it can be determined that the target protein has the disulfide bond cleavage activity.

The cleavage of the disulfide bond in the substrate protein can be confirmed by detecting a thiol group formed by the cleavage of the disulfide bond. For example, the thiol group may be detected by using a reagent that is specifically added to the thiol group, such as a maleimide compound. For example, maleimide PEG (polyethylene glycol with a maleimide group added) may be reacted with the substrate protein after the reaction, and SDS-polyacrylamide gel electrophoresis (PAGE) may be performed. The maleimide PEG specifically binds to the thiol group. Therefore, when the thiol group is formed by the cleavage of the disulfide bond, the maleimide PEG is added to the thiol group. Accordingly, a molecular weight of the substrate protein is increased compared to that when the disulfide bond is not cleaved. The cleavage of the disulfide bond can be detected based on the increase in the molecular weight of the substrate protein.

The disulfide bond cleavage enzyme is not particularly limited as long as one has the disulfide bond cleavage activity. Examples of the disulfide bond cleavage enzyme include a protein containing a C(X)ₙC motif (C represents a cysteine residue, X represents any amino acid residue, and n represents an integer of 2 to 10).

In a formation reaction of the disulfide bond, the C(X)ₙC motif in an oxidized state acts on the cysteine residue (C) of the substrate protein in an unfolded state to form the disulfide bond between the cysteine residues in the substrate protein. In this case, the C(X)ₙC motif is converted to a reduced state.

In a cleavage reaction of the disulfide bond, the reduced C(X)ₙC motif reduces the disulfide bond of the substrate protein in a folded state to cleave the disulfide bond. In this case, the C(X)ₙC motif is converted to the oxidized state.

The C(X)ₙC motif is preferably a CXXC motif, and more preferably a CGHC motif (C represents a cysteine residue, G represents a glycine residue, and H represents a histidine residue). Examples of a protein containing the CXXC motif include a protein containing a thioredoxin-like domain. The thioredoxin-like domain is a domain having a structure similar to that of thioredoxin and has a catalytic activity in the formation reaction and the cleavage reaction of the disulfide bond. The thioredoxin-like domain contains the CXXC motif in an active site.

Specific examples of the disulfide bond cleavage enzyme include PDI family proteins. The PDI family proteins represent a group of proteins having the thioredoxin-like domain. The PDI family proteins are localized in endoplasmic reticulum. Examples of the PDI family proteins include protein disulfide isomerase (PDI), ERp46 (thioredoxin domain containing 5; also referred to as "TXNDC5"), ERp57 (protein disulfide-isomerase like protein ERp57), ERp72 (protein disulfide isomerase family A member 4), P5 (protein disulfide isomerase family A member 6; also referred to as "PDIA6") , ERp18 (thioredoxin domain containing 12; also referred to as "TXNDC12"), ERdj5 (DnaJ heat shock protein family (Hsp40) member C10; also referred to as "DNAJC10"), PDIr (protein disulfide isomerase family A member 5; also referred to as "PDIA5"), and PDIp (protein disulfide isomerase family A member 2; also referred to as "PDIA2"). The disulfide bond cleavage enzyme is preferably the PDI family protein, more preferably at least one selected from the group consisting of PDI, ERp46, ERp57, ERp72, and P5, and furthermore preferably at least one selected from the group consisting of PDI and ERp46.

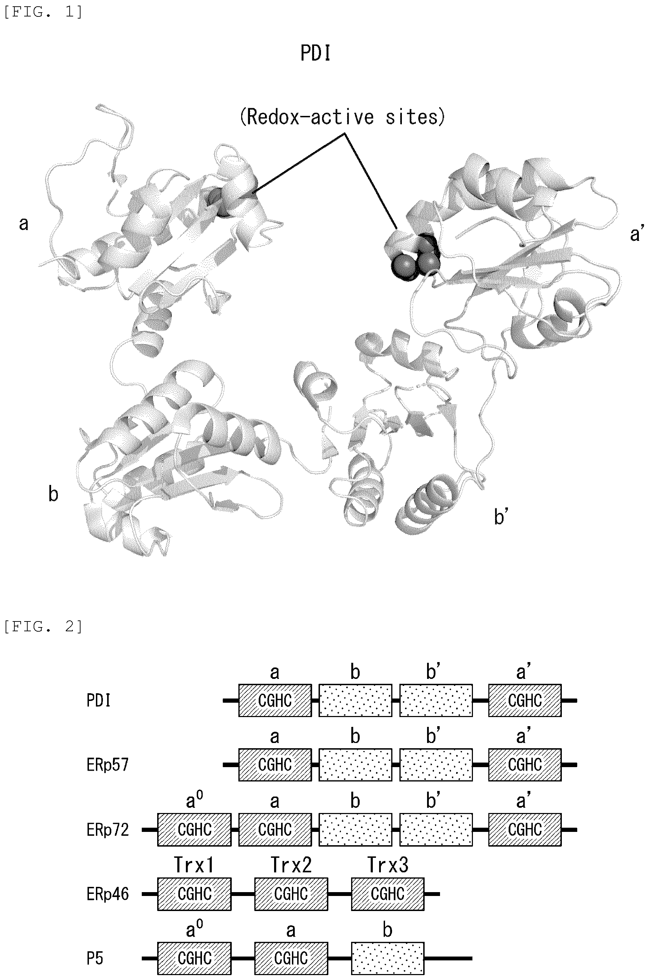

FIG. 1 shows a three-dimensional structure of PDI. PDI has four domains, that is, catalytic domains a and a' and non-catalytic domains b and b'. The catalytic domains a and a' are thioredoxin-like domains. Each of the a domain and the a' domain contains one redox-active site. One redox-active site contains one CGHC motif. FIG. 2 shows domain configurations of PDI, ERp57, ERp72, ERp46, and P5, which are the PDI family proteins. In the figure, "CGHC" indicates the CGHC motif, and indicates that the domain contains the CGHC motif.

An amino acid sequence of human PDI is shown as SEQ ID NO: 2. A nucleotide sequence encoding the amino acid sequence is shown as SEQ ID NO: 1. The human PDI may be a gene recombinant. An example of an amino acid sequence of the gene recombinant as the human PDI is shown as SEQ ID NO: 4 (nucleotide sequence: SEQ ID NO: 3).

An amino acid sequence of human ERp46 is shown as SEQ ID NO: 6. A nucleotide sequence encoding the amino acid sequence is shown as SEQ ID NO: 5. The human ERp46 may be a gene recombinant. An example of an amino acid sequence of the gene recombinant as the human ERp46 is shown as SEQ ID NO: 8 (nucleotide sequence: SEQ ID NO: 7).

An amino acid sequence of human ERp57 is shown as SEQ ID NO: 10. A nucleotide sequence encoding the amino acid sequence is shown as SEQ ID NO: 9. The human ERp57 may be a gene recombinant. An example of an amino acid sequence of the gene recombinant as the human ERp57 is shown as SEQ ID NO: 12 (nucleotide sequence: SEQ ID NO: 11).

An amino acid sequence of human ERp72 is shown as SEQ ID NO: 14. A nucleotide sequence encoding the amino acid sequence is shown as SEQ ID NO: 13. The human ERp72 may be a gene recombinant. An example of an amino acid sequence of the gene recombinant as the human ERp72 is shown as SEQ ID NO: 16 (nucleotide sequence: SEQ ID NO: 15).

An amino acid sequence of human P5 is shown as SEQ ID NO: 18. A nucleotide sequence encoding the amino acid sequence is shown as SEQ ID NO: 17. The human P5 may be a gene recombinant. An example of an amino acid sequence of the gene recombinant as the human P5 is shown as SEQ ID NO: 20 (nucleotide sequence: SEQ ID NO: 19).

Examples of an amino acid sequence of human ERp18 include one registered by GeneBank Accession No. NP_056997.1. Examples of the nucleotide sequence include one registered by GeneBank Accession No. NM_015913.4.

Examples of an amino acid sequence of human ERdj5 include one registered by GeneBank Accession No. NP_001258510.1. Examples of the nucleotide sequence include one registered by GeneBank Accession No. NM_001271581.3.

Examples of an amino acid sequence of human PDIr include one registered by GeneBank Accession No. NP_006801.1. Examples of the nucleotide sequence include one registered by GeneBank Accession No. NM_006810.4.

Examples of an amino acid sequence of human PDIp include one registered by GeneBank Accession No. NP_006840.2. Examples of the nucleotide sequence include one registered by GeneBank Accession No. NM_006849.4.

The PDI family protein may be a natural protein or a modified protein. Specific examples of the PDI family protein that can be used as the disulfide bond cleavage enzyme include the following polypeptides (A) to (C).

(A) Polypeptide containing an amino acid sequence of a natural PDI family protein (for example, SEQ ID NO: 2, 6, 10, 14, or 18).

(B) Polypeptide containing an amino acid sequence in which one or a plurality of amino acids are mutated in the amino acid sequence of the natural PDI family protein (for example, SEQ ID NO: 2, 6, 10, 14, or 18), and having a disulfide bond cleavage activity.

(C1) Polypeptide containing an amino acid sequence having 80% or more sequence identity with the amino acid sequence of the natural PDI family protein (for example, SEQ ID NO: 2, 6, 10, 14, or 18), and having a disulfide bond cleavage activity.

The polypeptide in (A) is a polypeptide consisting of the amino acid sequence of the natural PDI family protein, or a polypeptide obtained by adding the amino acid sequence to any one or both of an N-terminus and a C-terminus of the polypeptide. A length of the amino acid sequence added to the N-terminus or the C-terminus of the polypeptide is not particularly limited. Examples of the amino acid sequence to be added include an amino acid sequence of a peptide tag (FLAG tag, HA tag, 6×His tag, Myc tag, and the like), and an amino acid sequence of another protein. The number of amino acids in the amino acid sequence to be added is, for example, 2000 or less, 1000 or less, 500 or less, 400 or less, 300 or less, 200 or less, 100 or less, 50 or less, 30 or less, 20 or less, or 10 or less. The number of amino acids constituting the polypeptide in (A) is, for example, 4000 or less, 3000 or less, 2000 or less, 1000 or less, 800 or less, 700 or less, or 600 or less.

The polypeptide in (B) is a polypeptide consisting of an amino acid sequence in which one or a plurality of amino acids are mutated in the amino acid sequence of the natural PDI family protein, or a polypeptide obtained by adding an amino acid sequence to any one or both of the N-terminus and C-terminus of the polypeptide. Examples of the amino acid sequence to be added and the number of amino acids can be the same as those in (A) above.

The mutation of the amino acid may be any one of deletion, substitution, addition, and insertion, or a combination thereof. The number of mutated amino acids is not particularly limited. The number of mutated amino acids is, for example, 1 to 80, 1 to 60, 1 to 50, 1 to 40, 1 to 30, 1 to 20, 1 to 10, 1 to 5, 1 to 4, 1 to 3, 1, or 2. The "plurality of" refers to, for example, 2 to 80, 2 to 60, 2 to 50, 2 to 40, 2 to 30, 2 to 20, 2 to 10, 2 to 5, 2 to 4, 2 to 3, or 2. The number of mutated amino acids is preferably one or more. The "more" refers to 2 to 10, and may be 2 to 9, 2 to 8, 2 to 7, 2 to 6, 2 to 5, 2 to 4, 2 to 3, or 2.

A kind and a position of the mutated amino acid are not particularly limited. When the mutation is amino acid substitution, examples thereof include substitution of an original amino acid for an amino acid having similar properties. Properties of the amino acid may be determined by a side chain. For example, side chains of the original amino acid and the amino acid after the substitution may have similar properties. Examples of the amino acid substitution include conservative substitution. The conservative substitution is the amino acid substitution that does not significantly affect a function of a polypeptide. The amino acids can be classified, for example, according to the kind of side chain, into an acidic amino acid (aspartic acid, glutamic acid), a basic amino acid (lysine, arginine, histidine), and a neutral amino acid (amino acid with a hydrocarbon chain (glycine, alanine, valine, leucine, isoleucine, proline), an amino acid with a hydroxyl group (serine, threonine), an amino acid containing sulfur (cysteine, methionine), an amino acid with an amide group (asparagine, glutamine), an amino acid with an imino group (proline), and an amino acid with an aromatic group (phenylalanine, tyrosine, tryptophan)). Examples of the conservative substitution include substitution within these groups.

The polypeptide in (C) is a polypeptide consisting of an amino acid sequence having 80% or more sequence identity with the amino acid sequence of the natural PDI family protein, or a polypeptide obtained by adding an amino acid sequence to any one or both of the N-terminus and C-terminus of the polypeptide. Examples of the amino acid sequence to be added and the number of amino acids can be the same as those in (A) above.

The sequence identity is not particularly limited as long as the sequence identity is 80% or more. The sequence identity is, for example, 85% or more, 90% or more, 95% or more, 96% or more, 97% or more, 98% or more, 98.5% or more, 99% or more, and 99.5% or more.

The disulfide bond cleavage enzyme is not limited to the examples described above, and any one can be used. For example, the disulfide bond cleavage enzyme can use one registered in a public gene database or a public protein database such as GneneBank or UniProt without any particular limitation.

An organism from which the disulfide bond cleavage enzyme is derived is not particularly limited. From a viewpoint of avoiding an immune reaction to the disulfide bond cleavage enzyme, the disulfide bond cleavage enzyme is preferably derived from the same organism as an administration subject of the antiviral agent. For example, when the antiviral agent is administered to a human, it is preferable to use a disulfide bond cleavage enzyme derived from the human.

The disulfide bond cleavage enzyme can be obtained by a well-known method. When the disulfide bond cleavage enzyme is a natural protein, the disulfide bond cleavage enzyme may be purified from cells producing the disulfide bond cleavage enzyme by a well-known protein purification technique. Alternatively, a polynucleotide encoding the disulfide bond cleavage enzyme may be introduced into an appropriate host cell (for example, Escherichia coli or yeast) in an expressible state, and the disulfide bond cleavage enzyme may be produced in the host cell, and then the disulfide bond cleavage enzyme may be purified. Alternatively, the disulfide bond cleavage enzyme may be produced using a cell-free synthesis system.

When the antiviral agent contains the disulfide bond cleavage enzyme, the antiviral agent may contain one or two or more kinds of the disulfide bond cleavage enzyme.

### <Polynucleotide Containing Nucleotide Sequence Encoding Protein Having Disulfide Bond Cleavage Activity>

In one embodiment, the antiviral agent may contain a polynucleotide containing a nucleotide sequence encoding a protein having a disulfide bond cleavage activity (hereinafter, also referred to as a "disulfide bond cleavage enzyme coding sequence").

### (RNA Containing Disulfide Bond Cleavage Enzyme Coding Sequence)

The polynucleotide containing the disulfide bond cleavage enzyme coding sequence may be RNA. In this case, the polynucleotide is preferably an mRNA. In the case of the mRNA, the polynucleotide preferably includes a 5' untranslated region (5'UTR), a 3' untranslated region (3'UTR), a 5' terminal cap structure (5'Cap), and a 3' terminal poly A sequence in addition to the disulfide bond cleavage enzyme coding sequence. The mRNA may be a modified mRNA or a natural mRNA of the disulfide bond cleavage enzyme. Examples of the mRNA include the following.

(1) mRNA containing, in order from the 5' side, 5'Cap, 5'UTR, a disulfide bond cleavage enzyme coding region, 3'UTR, and poly A.
(2) mRNA containing, in order from the 5' side, 5'Cap, 5'UTR, a disulfide bond cleavage enzyme coding region, and poly A.
(3) mRNA containing, in order from the 5' side, 5'UTR, a disulfide bond cleavage enzyme coding region, 3'UTR, and poly A.
(4) mRNA containing, in order from the 5' side, 5'UTR, a disulfide bond cleavage enzyme coding region, and poly A.
(5) mRNA containing, in order from the 5' side, 5'Cap, 5'UTR, a disulfide bond cleavage enzyme coding region, and 3'UTR.
(6) mRNA containing, in order from the 5' side, 5'Cap, 5'UTR, and a disulfide bond cleavage enzyme coding region.
(7) mRNA containing, in order from the 5' side, 5'UTR, a disulfide bond cleavage enzyme coding region, and 3'UTR.
(8) mRNA containing, in order from the 5' side, 5'UTR and a disulfide bond cleavage enzyme coding region.

In (1) to (8) described above, the disulfide bond cleavage enzyme coding region is a region having a nucleotide sequence encoding the disulfide bond cleavage enzyme (hereinafter, also referred to as the "disulfide bond cleavage enzyme coding sequence"). The disulfide bond cleavage enzyme coding sequence may be identical to a coding sequence in an mRNA of a natural disulfide bond cleavage enzyme (for example, a PDI family protein). The disulfide bond cleavage enzyme coding sequence may have a mutation in the natural coding sequence as long as the disulfide bond cleavage enzyme coding sequence encodes a functional disulfide bond cleavage enzyme. For example, the disulfide bond cleavage enzyme coding sequence may have a silent mutation in the natural coding sequence. The disulfide bond cleavage enzyme coding sequence may be codon-optimized according to an application target organism. The disulfide bond cleavage enzyme coding sequence may have a high G/C content in order to stabilize the mRNA.

The mRNA of the disulfide bond cleavage enzyme can be produced by a well-known method. For example, the production can be performed by in vitro transcription of a template DNA encoding the disulfide bond cleavage enzyme. For example, the production can be performed according to a method described in Blood 108 (13) (2006)4009-17. Specifically, the production can be performed by cleaving, immediately downstream of a poly A/T chain, a template DNA in which the poly A/T chain is incorporated downstream of the disulfide bond cleavage enzyme coding sequence, performing in vitro transcription in a buffer solution containing a translation enzyme, a nucleotide, and a **5'** cap analog, and then purifying the mRNA.

A length of the poly A sequence of the mRNA of the disulfide bond cleavage enzyme is not particularly limited. The length of the poly A sequence can be, for example, 10 bases to 500 bases, preferably 30 bases to 300 bases, and more preferably 60 bases to 250 bases.

The sequences of the 5'UTR and the 3'UTR of the mRNA of the disulfide bond cleavage enzyme may have 100% sequence identity with naturally occurring sequences. Alternatively, the sequence of the 5'UTR may be partially or entirely substituted with a sequence of 5'UTR of an mRNA of another gene. The sequence of the 3'UTR may be partially or entirely substituted with a sequence of 3'UTR of an mRNA of another gene. Examples of the mRNA of another gene include, but are not limited to, an mRNA of a globin gene, an mRNA of a hydroxysteroid (17-β) dehydrogenase 4 gene, and an mRNA of an albumin gene.

Chemical modification of bases of an mRNA itself (coding region, 5'UTR, 3'UTR, poly A) may or may not be performed. If the chemical modification of the bases of the mRNA itself is not performed, a translation process is expected to be almost unimpaired because the mRNA itself is natural. If the chemical modification of the bases of the mRNA itself is performed, enzyme resistance of the mRNA can be improved and/or immunogenicity can be reduced. Examples of chemically modified bases of the mRNA itself include, but are not limited to, methylated bases (for example, 5-methylcytosine), sulfur-modified bases (for example, 2-thiouridine), pseudouridine, N1 methylpseudouridine, and 5 methoxyuridine.

Modification of a Cap structure of the mRNA may or may not be performed. The modification of the Cap structure is expected to stabilize the mRNA. Examples of the modification of the Cap structure include modification of a hydroxyl group at a 2nd position of a first or second ribose on a 5' side to a methoxy group. It is known that such modification improves expression efficiency.

### (DNA Containing Disulfide Bond Cleavage Enzyme Coding Sequence)

The polynucleotide containing the disulfide bond cleavage enzyme coding sequence may be DNA. In this case, the polynucleotide preferably contains an expression control sequence of the disulfide bond cleavage enzyme coding sequence in addition to the disulfide bond cleavage enzyme coding sequence. Examples of the expression control sequence include promoters, enhancers, poly A addition signals, and terminators. These expression control sequences may be any sequence that can function in cells of an administration subject of the antivirus. The disulfide bond cleavage enzyme coding sequence is preferably functionally linked to a promoter. The disulfide bond cleavage enzyme coding sequence is preferably functionally linked to an expression control sequence contained in the polynucleotide. The polynucleotide encoding the disulfide bond cleavage enzyme may be an expression vector for the disulfide bond cleavage enzyme.

When a subject is a mammal, specific examples of a promoter include, but are not limited to, a cytomegalovirus (CMV) promoter, an SRα promoter, an SV40 early promoter, a retroviral LTR, a rous sarcoma virus (RSV) promoter, a herpes simplex virus thymidine kinase (HSV-TK) promoter, an EF1α promoter, a metallothionein promoter, and a heat shock promoter.

When the expression vector is used, the kind of the vector is not particularly limited. The vector may be any vector that functions in cells of the administration subject of the antiviral agent. The vector may be a viral vector or a non-viral vector (plasmid, transposon vector, episomal vector, artificial chromosome vector, and the like).

Examples of viral vectors include a Sendai virus vector, a retrovirus (including lentivirus) vector, an adenovirus vector, an adeno-associated virus vector, a herpes virus vector, a vaccinia virus vector, a poxvirus vector, a poliovirus vector, a Silvis virus vector, a rhabdovirus vector, a paramyxovirus vectors, and an orthomyxovirus vector. The viral vector may be a replication-deficient viral vector that is replication-deficient.

Examples of plasmid vectors include pA1-11, pXT1, pRc/CMV, pRc/RSV, and pcDNAI/Neo.

Examples of an episomal vector include a vector that includes a sequence required for autonomous replication as a vector element. The sequence required for autonomous replication may be derived from Epstein-Barr virus (EBV) or Simian virus 40 (SV40). Specific examples of the vector element required for autonomous replication include an origin of replication and a gene that encodes a protein binding to the origin of replication and controlling replication. Examples include an origin of replication oriP and an EBNA-1 gene in EBV, and an origin of replication ori and an SV40LT gene in SV40.

Examples of an artificial chromosome vector include human artificial chromosome (HAC), a yeast artificial chromosome (YAC) vector, a bacterial artificial chromosome (BAC) vector, and P1-derived artificial chromosome (PAC).

### (Disulfide Bond Cleavage Enzyme Coding Sequence)

The disulfide bond cleavage enzyme coding sequence is not particularly limited as long as the sequence encodes the disulfide bond cleavage enzyme. Examples of the disulfide bond cleavage enzyme encoded by the disulfide bond cleavage enzyme coding sequence are the same as those described above. The disulfide bond cleavage enzyme coding sequence may encode a natural disulfide bond cleavage enzyme or may encode a modified disulfide bond cleavage enzyme. The disulfide bond cleavage enzyme coding sequence may include an intron, or may be a gene sequence on a genome of a natural disulfide bond cleavage enzyme. Alternatively, the disulfide bond cleavage enzyme coding sequence may not contain an intron. Examples of the disulfide bond cleavage enzyme coding sequence that does not contain an intron include the nucleotide sequences set forth in SEQ ID **NO:** 1 (human PDI), SEQ ID NO: 3 (human PDI recombinant), SEQ ID NO: 5 (human ERp46), SEQ ID NO: 7 (human ERp46 recombinant), SEQ ID NO: 9 (human ERp57), SEQ ID NO: 11 (human ERp57 recombinant), SEQ ID NO: 13 (human ERp72), SEQ ID NO: 15 (human ERp72 recombinant), SEQ ID NO: 17 (human P5), or SEQ ID NO: 19 (human P5 recombinant). When the polynucleotide containing the disulfide bond cleavage enzyme coding sequence is RNA, the nucleotide sequence set forth in the SEQ ID NO is translated to a nucleotide sequence in which "T" is replaced with "U".

Examples of the disulfide bond cleavage enzyme coding sequence include a PDI family protein coding sequence. Specific examples of the PDI family protein coding sequence include the following (a) to (c).
(a) Nucleotide sequence encoding a natural PDI family protein (for example, SEQ ID NOs: 1, 5, 9, 13, and 17).
(b) Nucleotide sequence in which one or a plurality of nucleotides are mutated in the nucleotide sequence encoding the natural PDI family protein (for example, SEQ ID NOs: 1, 5, 9, 13, and 17), and which encodes a polypeptide having the disulfide bond cleavage activity.
(c) Nucleotide sequence having 80% or more sequence identity with a polynucleotide consisting of the nucleotide sequence encoding the natural PDI family protein (for example, SEQ ID NOs: 1, 5, 9, 13, and 17), and which encodes the polypeptide having the disulfide bond cleavage activity.

In the nucleotide sequence in (b), the mutation of the nucleotide may be any one of deletion, substitution, addition, and insertion, or a combination thereof. The number of mutated nucleotides is not particularly limited, as long as the polypeptide encoded by the resulting nucleotide sequence has the disulfide bond cleavage activity. The number of mutated nucleotides is, for example, 1 to 300, 1 to 200, 1 to 150, 1 to 120, 1 to 100, 1 to 80, 1 to 60, 1 to 50, 1 to 40, 1 to 30, 1 to 20, 1 to 10, 1 to 5, 1 to 4, 1 to 3, 1, or 2. The "plurality of" refers to, for example, 2 to 300, 2 to 200, 2 to 150, 2 to 120, 2 to 100, 2 to 80, 2 to 60, 2 to 50, 2 to 40, 2 to 30, 2 to 20, 2 to 10, 2 to 5, 2 to 4, 2 to 3, or 2. The number of mutated nucleotides is preferably one or more. The "more" refers to 2 to 10, and may be 2 to 9, 2 to 8, 2 to 7, 2 to 6, 2 to 5, 2 to 4, 2 to 3, or 2.

In the nucleotide sequence in (c), the sequence identity is not particularly limited as long as the sequence identity is 80% or more. The sequence identity is, for example, 85% or more, 90% or more, 95% or more, 96% or more, 97% or more, 98% or more, 98.5% or more, 99% or more, and 99.5% or more.

In the disulfide bond cleavage enzyme coding sequence, a degenerate codon may be one that is frequently used in an administration subject of the antiviral agent. For example, the codon of the disulfide bond cleavage enzyme coding sequence may be optimized according to the administration subject of the antiviral agent. The mutation of the nucleotide may be a genetic mutation (silent mutation) that does not change the amino acid sequence of the encoded protein.

When the antiviral agent contains a polynucleotide containing the disulfide bond cleavage enzyme coding sequence, the antiviral agent may contain one or two or more kinds of the polynucleotide.

### <Application Subject>

A virus to which the antiviral agent is applied is not particularly limited. The antiviral agent is preferably applied to a virus that contains a protein folded by a disulfide bond, for example. The protein containing a disulfide bond is not particularly limited, and is preferably at least one protein selected from the group consisting of a spike protein, an envelope protein, and a capsid protein. A spike is a structure protruding from an outer surface of the virus. The spike is generally present in an envelope and is involved in entry of virus particles into host cells. The spike protein is a protein that constitutes the spike. The envelope is a lipid bilayer membrane structure located at an outermost side of a virus particle. The envelope protein is a protein that constitutes the envelope. A capsid is a protein shell that surrounds viral genome. The capsid protein is a protein that constitutes the capsid. In particular, it is preferable that the spike protein contains a disulfide bond. It is more preferable that the spike protein contains a disulfide bond in a receptor binding domain (RBD). In one embodiment, the antiviral agent is preferably applied to a virus that contains a disulfide bond in at least one protein selected from the group consisting of the spike protein, the envelope protein, and the capsid protein, more preferably applied to a virus that contains the disulfide bond in the spike protein, and more preferably applied to a virus that contains the disulfide bond in the RBD.

Examples of the virus to which the antiviral agent is applied include coronavirus, influenza virus, RS virus, Ebola virus, human immunodeficiency virus, rotavirus, Zika virus, dengue virus, Japanese encephalitis virus, hepatitis virus (hepatitis A virus, hepatitis B virus, hepatitis C virus, hepatitis D virus, hepatitis E virus), and papilloma virus. Among these, since the coronavirus has the disulfide bond in the RBD, the coronavirus is preferred as the virus to which the antiviral agent is applied.

The "coronavirus" refers to a virus belonging to coronaviridae. The coronaviridae is further divided into coronavirinae (orthocoronavirinae) and torovirinae. The coronavirinae is further divided into four genera, that is, Alpha (α)coronavirus, Beta (β)coronavirus, Gamma (y)coronavirus, and Delta (δ)coronavirus. The coronavirus as an application subject may be any of these. Specific examples of the coronaviruses as the application subject include, but are not limited to, SARS-CoV-2, SARS-CoV-1, MERS-CoV, HCoV-HKU1, HCoV-OC43, HCoV-229E, and HCoV-NL63. A virus as the application subject may be a mutant strain. Examples of a mutant strain of SARS-CoV-2 include, but are not limited to, a Delta strain and an Omicron strain.

The administration subject of the antiviral agent is not particularly limited. The application subject of the antiviral agent is preferably a eukaryote, and may be an animal or a plant. Among these, the application subject of the antiviral agent is more preferably a mammal. The mammal may be a human or a mammal other than a human. Examples of the mammal other than a human include, but are not limited to, primates (monkeys, chimpanzees, gorillas, or the like), rodents (mice, hamsters, rats, or the like), rabbits, dogs, cats, cows, goats, sheep, and horses.

The antiviral agent of the present embodiment contains the disulfide bond cleavage enzyme, and the enzyme cleaves the disulfide bond of the protein that constitutes a virus, thereby inactivating the virus. For example, if the virus contains the disulfide bond in the RBD, the RBD is unfolded by cleaving the disulfide bond in the RBD. The disulfide bond cleavage enzyme has a binding activity to the protein containing the disulfide bond, and thus the disulfide bond cleavage enzyme can competitively inhibit the binding of the RBD containing the disulfide bond to a receptor of a host cell. Accordingly, it is difficult for the virus to bind to the receptor of the host cell. As a result, infection of the host cell by the virus is prevented.

The antiviral agent of the present embodiment exerts an antiviral activity by inactivating a virus protein through cleavage of the disulfide bond, and is therefore not susceptible to the mutation in the virus protein. Therefore, the antiviral agent can be applied to a wide range of viruses. A risk of side effects can be reduced by using a disulfide bond cleavage enzyme derived from the same biological species as that of the administration subject.

### [Pharmaceutical Composition]

A second aspect of the invention is a pharmaceutical composition for treating or preventing a virus infection. In one embodiment, the pharmaceutical composition contains the antiviral agent according to the first aspect and a pharmaceutically acceptable carrier. The pharmaceutical composition of the present embodiment contains the antiviral agent according to the first aspect as an active ingredient.

The "pharmaceutically acceptable carrier" refers to a carrier that does not inhibit a physiological activity of the active ingredient and does not show substantial toxicity to the administration subject. The expression "not show substantial toxicity" refers to that an ingredient of the carrier does not show toxicity to the administration subject at a dose that is normally used. In the pharmaceutical composition of the present embodiment, the pharmaceutically acceptable carrier is a carrier that does not inhibit the antiviral activity of the antiviral agent according to the first aspect and does not show the substantial toxicity to the administration subject. The pharmaceutically acceptable carrier includes any well-known pharmaceutically acceptable ingredient that is typically considered to be an inactive ingredient. Examples of the pharmaceutically acceptable carrier include, but are not limited to, solvents, diluents, vehicles, excipients, glidants, binders, granulating agents, dispersing agents, suspending agents, wetting agents, lubricants, disintegrants, solubilizers, stabilizers, emulsifiers, and fillers. The pharmaceutically acceptable carrier may be used alone or in combination of two or more kinds thereof.

The pharmaceutical composition may contain other ingredients in addition to the above ingredient. The other ingredients are not particularly limited, and can use those commonly used in the pharmaceutical field without any particular limitation. Examples of the other ingredients include a pharmaceutical additive other than those described above. Examples of the pharmaceutical additive include, but are not limited to, preservatives (for example, antioxidants), chelating reagents, flavoring agents, sweeteners, thickeners, buffers, and colorants. The pharmaceutical composition may contain an active ingredient other than the antiviral agent according to the first aspect. Examples of the active ingredient include, but are not limited to, other antiviral agents, antibiotics, antiinflammatory agents, antipyretics, and analgesics. When the antiviral agent includes a polynucleotide containing a disulfide bond cleavage enzyme coding sequence, the pharmaceutical composition may include a transfection reagent. Examples of the transfection reagent include, but are not limited to, polyethyleneimine, cationic lipids, cationic liposomes, and cationic polymers. The other ingredients may be used alone or in combination of two or more kinds thereof.

A dosage form of the pharmaceutical composition is not particularly limited, and may be a dosage form generally used as a pharmaceutical formulation. The pharmaceutical composition of the present embodiment may be an oral formulation or a parenteral formulation, and is preferably the parenteral formulation. Examples of the oral formulation include, for example, tablets, coated tablets, pills, powders, granules, capsules, syrups, fine granules, liquids, drop loves, and emulsions. Examples of the parenteral formulation include injections, suppositories, nasal drops, enteral formulations, and inhalants. The pharmaceutical compositions of these dosage forms can be formulated according to standard methods (for example, methods described in Japanese Pharmacopoeia). The pharmaceutical composition is preferably a parenteral formulation, and more preferably an injection.

An administration route of the pharmaceutical composition of the present embodiment is not particularly limited, and may be oral or parenteral administration, and is preferably the parenteral administration. As the administration route of the parenteral administration, examples of the parenteral administration include intravenous administration, intranasal administration, intratracheal administration, intrapulmonary administration, subcutaneous administration, intradermal administration, intramuscular administration, intraperitoneal administration, and enteral administration. When the antiviral agent contains the disulfide bond cleavage enzyme, the administration route is preferably the intravenous administration. When the antiviral agent contains the polynucleotide containing the disulfide bond cleavage enzyme coding sequence, the administration route is preferably the subcutaneous administration, the intradermal administration, or the intramuscular administration.

The pharmaceutical composition can be administered in a therapeutically effective amount of the antiviral agent according to the first aspect. The "therapeutically effective amount" means an amount of a drug effective for treating or preventing a target disease. For example, the therapeutically effective amount of the antiviral agent can be an amount effective for preventing a virus infection. The therapeutically effective amount may be appropriately determined depending on symptoms, body weight, age, and gender of a patient, and the dosage form and an administration method of the pharmaceutical composition. For example, the pharmaceutical composition can be administered in a range of 0.01 mg to 1000 mg per kg of the body weight of the administration subject as a single dose of the antiviral agent. The dose may be 0.05 mg/kg to 500 mg/kg, 0.1 mg/kg to 300 mg/kg, 0.2 mg/kg to 200 mg/kg, or 0.3 mg/kg to 100 mg/kg.

The pharmaceutical composition may contain a therapeutically effective amount of the antibody per unit dosage form. For example, a content of the antiviral agent in the pharmaceutical composition may be 0.01% by mass to 90% by mass, 0.05% by mass to 80% by mass, or 0.1% by mass to 60% by mass.

The pharmaceutical composition may be administered in a single dose or repeatedly. In the case of repeated administration, an administration interval may be appropriately determined depending on symptoms, body weight, age, and gender of a patient, and the dosage form and the administration method of the pharmaceutical composition. The administration interval may be, for example, every few hours, 2 times to 3 times a day, once a day, once every 2 days to 3 days, once a week, once a month, and once every few months.

The pharmaceutical composition is used to treat or prevent a virus infection. The virus infection as an application subject is not particularly limited. In the virus infection, for example, it is preferable that a virus containing a protein folded by a disulfide bond is a causative virus. The virus infection is preferably caused by, for example, a causative virus that contains a spike protein having a disulfide bond. Examples of the virus infection include a coronavirus infection, influenza (influenza virus infection), an RS virus infection, dengue fever (dengue virus infection), Ebola hemorrhagic fever (Ebola virus infection), a human immunodeficiency virus infection (AIDS), a rotavirus infection, Zika fever (Zika virus infection), Japanese encephalitis (Japanese encephalitis virus infection), viral hepatitis (hepatitis virus infection), and a papillomavirus infection. The virus infection is preferably caused by a causative virus that contains a disulfide bond in a receptor binding domain (RBD). Examples of such a virus infection include the coronavirus infection. Specific examples of the coronavirus infection include, but are not limited to, COVID-19, SARS, MERS, and a seasonal coronavirus infection.

The administration subject of the pharmaceutical composition is not particularly limited. The administration subject of the pharmaceutical composition is preferably a eukaryote, and may be an animal or a plant. Among these, the administration subject of the pharmaceutical composition is more preferably a mammal. The mammal may be a human or a mammal other than a human. Examples of the mammal other than a human are the same as those exemplified for the antiviral agent.

### [Virus Infection Severity Risk Evaluation Kit]

A third aspect of the invention is a virus infection severity risk evaluation kit. In one embodiment, the virus infection severity risk evaluation kit contains a specific binding substance for a protein having a disulfide bond cleavage activity (disulfide bond cleavage enzyme).

The disulfide bond cleavage enzyme is a protein involved in protein folding and degradation, and is found in a wide range of organisms. In mammals, some of the protein cleavage enzymes are secreted outside cells and are also present in body fluids. It is considered that the disulfide bond cleavage enzymes in the body fluids act on viruses present in the body fluids and cleave disulfide bonds in virus proteins to inactivate the viruses. If a concentration of the disulfide bond cleavage enzymes in the body fluids is lower than that of a healthy individual, the viruses are not inactivated and grow, increasing a risk of the virus infection becoming severe. Therefore, by measuring a concentration of a disulfide bond cleavage enzyme in a body fluid of a test subject, a severity risk of a virus infection can be evaluated. It is considered that the disulfide bond cleavage enzyme present in a cell acts on a virus that enters the cell, and cleaves the disulfide bond of the viral protein to inactivate the virus. Therefore, by measuring the concentration of the disulfide bond cleavage enzyme in a biological sample (body fluid, tissue fragment, or the like) obtained from the test subject, the severity risk of the virus infection can be evaluated.

### <Disulfide Bond Cleavage Enzyme>

Examples of the disulfide bond cleavage enzyme to which the specific binding substance binds are the same as those described above. The disulfide bond cleavage enzyme is preferably a PDI family protein, more preferably at least one selected from the group consisting of PDI, ERp46, ERp57, ERp72, and P5, and furthermore preferably at least one selected from the group consisting of PDI and ERp46.

### <Specific Binding Substance>

In one embodiment, the kit includes the specific binding substance for the disulfide bond cleavage enzyme. The "specific binding substance" refers to a substance that has an activity (specific binding properties) of specifically binding to a substance to be measured. Specific binding to the substance to be measured means that the substance has a high binding affinity to the substance to be measured, but does not bind to or barely binds to other substances. The specific binding substance for the disulfide bond cleavage enzyme is a substance that has specific binding properties to the disulfide bond cleavage enzyme.

Examples of the specific binding substance include antibodies and aptamers.

An intact antibody is unnecessary as long as the antibody has an antigen binding activity, and the antibody may be an antigen binding fragment. The "antigen binding fragment" is a polypeptide that contains a part of the antibody and maintains the antigen binding activity of the original antibody. The antigen binding fragment preferably contains all six complementarity determining regions (CDRs) of the original antibody. That is, the antigen binding fragment preferably contains all of CDR1, CDR2, and CDR3 of a heavy chain variable region, and CDR1, CDR2, and CDR3 of a light chain variable region. Examples of the antigen binding fragment include Fab, Fab', F(ab')₂, a variable region fragment (Fv), a disulfide bond Fv, a single chain Fv (scFv), and sc(Fv)₂.

The antibody may be derived from any organism. Examples of the organism from which the antibody is derived include, but are not limited to, mammals (human, mice, rats, rabbits, horses, cows, pigs, monkeys, dogs, and the like), and birds (chickens, ostriches).

The antibody may be any class and subclass of immunoglobulin. The antibody may be a monoclonal antibody or a polyclonal antibody.

The antibody can be produced by a well-known method such as immunization, hybridoma, and phage display.

An aptamer is a substance that has specific binding properties to a target substance. Examples of the aptamer include a nucleic acid aptamer and a peptide aptamer. The nucleic acid aptamer can be selected by, for example, systematic evolution of ligand by exponential enrichment (SELEX). The peptide aptamer can be selected by, for example, a two-hybrid method using yeast.

The specific binding substance may be an antibody, an antigen binding fragment, or an aptamer, and is preferably an antibody. The specific substance for the disulfide bond cleavage enzyme may be an antibody or an antigen binding fragment for the disulfide bond cleavage enzyme, or an aptamer for the disulfide bond cleavage enzyme.

The specific binding substance contained in the kit may be one, or two or more kinds thereof. When the kit contains two or more kinds of the specific binding substance, each specific binding substance may have specific binding properties for a different disulfide bond cleavage enzyme.

The specific binding substance may be immobilized on a solid phase carrier. Examples of the solid phase carrier include a well plate (for example, a 96-well microplate), a membrane (for example, a nitrocellulose membrane and a poly vinylidene fluoride membrane), a slide glass, a magnetic bead, and a latex particle. A method for immobilizing the specific binding substance on the solid phase carrier is not particularly limited. A well-known method can be appropriately selected depending on a material of the solid phase carrier.

When the specific binding substance is immobilized on the solid phase carrier, the kit may contain a specific binding substance (secondary specific binding substance) binding to the disulfide bond cleavage enzyme at an epitope which is different from that of the specific binding substance (primary specific binding substance) immobilized on the solid phase carrier.

For example, the kit may contain, as the specific binding substance for the disulfide bond cleavage enzyme, the primary specific binding substance immobilized on the solid phase carrier and the secondary specific binding substance not immobilized on the solid phase carrier.

The specific binding substance for the disulfide bond cleavage enzyme may be labeled. A labeling substance for labeling is not particularly limited, and can use well-known substances. Examples of the labeling substance include an enzyme label such as peroxidase (for example, horseradish peroxidase) and alkaline phosphatase; a fluorescent label such as carboxyfluorescein (FAM), 6-carboxy-4',5'-dichloro-2',7'-dimethoxyfluorescein (JOE), fluorescein isothiocyanate (FITC), tetrachlorofluorescein (TET), 5'-hexachloro-fluorescein-CE phosphoramidite (HEX), Cy3, Cy5, Alexa568, and Alexa647; a radioisotope label such as iodine-125; an electrochemiluminescent label such as a ruthenium complex; biotin; and a metal nanoparticle. The labeling of the specific binding substance can be performed by appropriately selecting a well-known method depending on the kind of the labeling substance. When biotin is used as the labeling substance, the kit may contain labeled avidin or an avidin derivative (streptavidin or the like). Examples of the labeling substance for avidin or a derivative thereof include a labeling substance other than the biotin among the labeling substances exemplified above.

When the kit contains the secondary specific binding substance, the secondary specific binding substance is preferably labeled.

### <Other Elements>

The kit may contain other elements in addition to the specific binding substance for the disulfide bond cleavage enzyme. Examples of the other elements include a detection reagent for the labeling substance, a standard sample of the disulfide bond cleavage enzyme, a biological sample preparation reagent, a diluent, buffers, a solid phase carrier, and an instruction manual.

The detection reagent for the labeling substance is a reagent for detecting the labeling substance used to label the specific binding substance. For example, when the labeling substance is an enzyme label, the detection reagent contains a substrate for the enzyme. For example, when the enzyme label is peroxidase, examples of the detection reagent include 3,3',5,5'-tetramethylbenzidine (TMB), o-phenylenediamine dihydrochloride (OPD), and 2,2'-azino-di-[3-ethyl-benzothiazoline-6 sulfonic acid] diammonium salt (ABTS). When the enzyme label is alkaline phosphatase, examples of the detection reagent include p-nitrophenyl-phosphate (pNPP).

The standard sample of the disulfide bond cleavage enzyme is a reagent for creating a standard curve of the disulfide bond cleavage enzyme. The standard sample of the disulfide bond cleavage enzyme can be used for a purified product of the disulfide bond cleavage enzyme to be measured. For example, when a PDI family protein is to be measured, a purified product of the PDI family protein (PDI, ERp46, ERp57, ERp72, P5, or the like) can be used.

The biological sample preparation reagent is a reagent for preparing a biological sample collected from a test subject. Examples of the biological sample to which the test kit of the present embodiment is applied include a blood sample, urine, lymph, a tissue fluid, saliva, a pharyngeal swab, a nasal swab, and a tissue fragment. Examples of the blood sample include plasma and serum. The biological sample preparation reagent is a reagent for adjusting a biological sample to be a sample suitable for measuring the disulfide bond cleavage enzyme. For example, when the biological sample is a blood sample, examples of the blood sample preparation reagent include a reagent for preparing a plasma sample or a serum sample from blood collected from a test subject. Examples of such a reagent include heparin, EDTA, and sodium citrate. Examples of the biological sample preparation reagent include a diluent for the biological sample. Examples of the diluent include a well-known buffer solution such as a phosphate buffer solution, a Tris buffer solution (Tri-HCl or the like), and phosphate buffered saline (PBS).

Examples of the buffers include a buffer used in a binding reaction between the disulfide bond cleavage enzyme and the specific binding substance, and a buffer used in a detection reaction of the labeling substance. Specific examples of the buffers include, for example, a blocking buffer and a washing buffer. These buffers can be any buffer that is generally used in immunoassays and the like without any particular limitation.

The solid phase carrier is used to immobilize the specific binding substance for the disulfide bond cleavage enzyme. Examples of the solid phase carrier include those exemplified above.

The kit may be an ELISA kit. In the case of the ELISA kit, for example, a primary specific binding substance immobilized on a solid phase carrier, a labeled secondary specific binding substance, and a detection reagent for the labeling substance may be contained. The ELISA kit may further contain a biological sample preparation reagent, an oxidized fatty acid extraction reagent, a diluent, a standard sample of the disulfide bond cleavage enzyme, and buffers (blocking buffer, washing buffer, or the like).

The kit can be used in the virus infection severity risk evaluation method described below.

### [Virus Infection Severity Risk Evaluation Method]

A fourth aspect of the invention is the virus infection severity risk evaluation method. In one embodiment, the virus infection severity risk evaluation method includes a step (hereinafter, also referred to as "step (a)") of measuring a concentration of a protein having a disulfide bond cleavage activity in a biological sample from a test subject.

<Virus Infection to Be Evaluated for Severity Risk>

In the virus infection to be evaluated for a severity risk, for example, it is preferable that a virus containing a protein folded by a disulfide bond is a causative virus. Examples of the virus infection include a coronavirus infection, influenza (influenza virus infection), an RS virus infection, dengue fever (dengue virus infection), Ebola hemorrhagic fever (Ebola virus infection), a human immunodeficiency virus infection (AIDS), a rotavirus infection, Zika fever (Zika virus infection), Japanese encephalitis (Japanese encephalitis virus infection), viral hepatitis (hepatitis virus infection), and a papillomavirus infection. The virus infection is preferably caused by a causative virus that contains a disulfide bond in a receptor binding domain (RBD). Examples of such a virus infection include COVID-19, SARS, MERS, and a seasonal coronavirus infection.

### <Biological Sample from Test Subject>

The "test subject" refers to a subject whose severity risk of the virus infection is evaluated by an evaluation method of the present embodiment. The test subject may be a subject suffering from the virus infection, or a subject at high risk of suffering from the virus infection. Examples of the test subject are the same as the administration subject of the antiviral agent.

The "biological sample" refers to a sample containing at least a part of body fluid components and/or cellular components of the test subject. The biological sample from the test subject can be obtained by collecting a body fluid or tissue of the test subject. Examples of the biological sample from the test subject include, but are not limited to, a blood sample, urine, lymph, a tissue fluid, saliva, a pharyngeal swab, a nasal swab, and a tissue fragment. The biological sample may be prepared by appropriately processing the body fluid or tissue fragment collected from the test subject.

The "blood sample" refers to a sample containing at least a part of blood components, and may be any of whole blood, serum, and plasma, or may be obtained by diluting these. The blood sample is preferably serum or plasma.

The blood sample of the test subject is a sample containing blood components collected from the test subject, and is preferably plasma or serum prepared from the blood collected from the test subject. The method for preparing plasma or serum from the blood is not particularly limited, and any well-known method may be used. Examples of a method for separating plasma from the blood include a method of adding heparin, EDTA, sodium citrate, and the like to the blood to perform centrifugation. In order to prevent decomposition of the disulfide bond cleavage enzyme, the centrifugation is preferably performed under cooling (for example, 4°C).

### <Measurement Step: Step (a)>

The method for measuring the concentration of the disulfide bond cleavage enzyme in the biological sample from the test subject is not particularly limited, and any well-known method can be used.

Examples of the method for measuring the disulfide bond cleavage enzyme include a method using a specific binding substance for the disulfide bond cleavage enzyme.

For example, the biological sample from the test subject is brought into contact with the specific binding substance for the disulfide bond cleavage enzyme bound to the solid phase carrier. Accordingly, the disulfide bond cleavage enzyme binds to the specific binding substance, and the disulfide bond cleavage enzyme in the biological sample is captured on the solid phase carrier. Next, the labeled secondary specific binding substance is brought into contact with the solid phase carrier. Accordingly, the secondary specific binding substance binds to the disulfide bond cleavage enzyme captured on the solid phase carrier. Next, the disulfide bond cleavage enzyme in the biological sample can be measured by measuring the labeling substance that binds to the secondary specific binding substance.

Specific examples of the method for measuring the disulfide bond cleavage enzyme using the specific binding substance include enzyme immunoassays (EIA, ELISA), radioimmunoassays (RIA), fluorescent enzyme immunoassays (FLEIA), chemiluminescent enzyme immunoassays (CLEIA), chemiluminescent immunoassays (CLIA), electrochemiluminescent immunoassays (ECLIA), and fluorescent antibody assays (FA).

The disulfide bond cleavage enzyme measured in this step may be one or two or more kinds thereof. Examples of the disulfide bond cleavage enzyme to be measured are the same as those described above, and the PDI family protein (PDI, ERp46, ERp57, ERp72, P5, or the like) is preferred.

### <Other Steps>

The method may include other steps in addition to the measurement step (step (a)). Examples of the other steps include a step of evaluating the severity risk of the virus infection based on the concentration of the disulfide bond cleavage enzyme measured in the measurement step, and a step of treating or preventing the virus infection based on the evaluation result.

### <<Evaluation Step: Step (b)>>

In one embodiment, the method may include the step of evaluating the severity risk of the virus infection based on the concentration of the disulfide bond cleavage enzyme measured in the measurement step.

For example, if the concentration of the disulfide bond cleavage enzyme in the biological sample is lower than a reference value, the test subject can be evaluated as one having a high risk of the virus infection becoming severe. Alternatively, for example, if the concentration of the disulfide bond cleavage enzyme in the biological sample is equal to or higher than the reference value, the test subject can be evaluated as one having a low risk of the virus infection becoming severe.

The reference value may be, for example, a numerical value calculated by performing statistical processing or the like from a concentration of a disulfide bond cleavage enzyme in a biological sample measured in a group of any number of healthy people. The reference value may be, for example, a numerical value calculated by performing statistical processing or the like from a concentration of a disulfide bond cleavage enzyme in a biological sample measured in a group of any number of people infected with, affected by, and/or recovered from a virus infection. The reference value may be, for example, a numerical value calculated by performing statistical processing or the like from a concentration of a disulfide bond cleavage enzyme in a biological sample measured in a group of people with mild virus infection. The reference value may be, for example, a numerical value calculated by performing statistical processing or the like from a concentration of a disulfide bond cleavage enzyme in a biological sample measured in a group of people with severe virus infection.

### <<Treatment/Prevention Step: Step (c)>>

In one embodiment, the method may include the step of treating or preventing the virus infection based on the evaluation of the severity risk performed in the evaluation step (step (b)).

The treatment method or prevention method can be appropriately selected depending on the kind of the virus infection and the evaluation of the severity risk in the evaluation step. Examples of the treatment method or prevention method include administrating the antiviral agent.

For example, if the severity risk is evaluated to be high in the evaluation step, the disulfide bond cleavage enzyme may be administered to the test subject. Therefore, the method may include a step of administering the disulfide bond cleavage enzyme to the test subject evaluated as one having a high severity risk in the evaluation step. The disulfide bond cleavage enzyme administered to the test subject may be in the form of the antiviral agent according to the first aspect, or the pharmaceutical composition according to the second aspect.

In one embodiment, the method may include a step of determining the test subject to be administered with the antiviral agent based on the evaluation of the severity risk performed in the evaluation step (step (b)). For example, in one embodiment, the method may be a method for determining the test subject to be administered with the antiviral agent, and the determination method includes the following steps.

A step (A) of measuring a concentration of a protein having a disulfide bond cleavage activity in a biological sample from a test subject,
a step (B) of evaluating a severity risk of a virus infection in the test subject based on the concentration of the protein having the disulfide bond cleavage activity; and
a step (C) of determining whether the test subject determined to have a high severity risk is a test subject to be administered with an antiviral agent.

The step (A) is the same as the step (a) described above. The step (B) is the same as the step (b) described above. The antiviral agent determined to be administered in the step (c) can be appropriately selected depending on the kind of virus. Examples of the antiviral agent include the antiviral agent according to the first aspect.

In one embodiment, the present disclosure provides a method for identifying a biological sample derived from a test subject having a high severity risk of a virus infection, the method including a step (A1) of measuring a concentration of a protein having a disulfide bond cleavage activity in the biological sample from the test subject, and a step (B1) of identifying a biological sample in which the concentration of the protein having the disulfide bond cleavage activity is lower than a reference value as the biological sample derived from the test subject having the high severity risk of the virus infection.

The step (A1) is the same as the step (a) described above. The reference value in the step (B1) is the same as that in the step (b) described above. Examples of the virus infection as the application subject are the same as those described above.

In one embodiment, the present disclosure provides a marker for determining the severity risk of the virus infection, the marker containing the protein having the disulfide bond cleavage activity.

According to the evaluation method of the present aspect, in the biological sample from the test subject, the severity risk of the virus infection can be evaluated by a simple method of measuring the concentration of the disulfide bond cleavage enzyme in the biological sample.

### [Other Embodiments]

In one embodiment, the present disclosure provides a method for treating or preventing a virus infection, the method including a step of administering, to a subject, at least one selected from the group consisting of (a) a protein having a disulfide bond cleavage activity, and (b) a polynucleotide containing a nucleotide sequence encoding the protein.

In one embodiment, the present disclosure provides at least one selected from the group consisting of (a) a protein having a disulfide bond cleavage activity, and (b) a polynucleotide containing a nucleotide sequence encoding the protein, for use in treating or preventing a virus infection.

In one embodiment, the present disclosure provides a use of at least one selected from the group consisting of (a) a protein having a disulfide bond cleavage activity, and (b) a polynucleotide containing a nucleotide sequence encoding the protein in production of a pharmaceutical composition for treating or preventing a virus infection.

Examples of the protein having the disulfide bond cleavage activity (disulfide bond cleavage enzyme) are the same as those described above, and specific examples thereof include a PDI family protein (PDI, ERp46, ERp57, ERp72, P5, or the like). Examples of the virus infection are the same as those described above.

### Examples

The invention will be described below with reference to Examples, and the invention is not limited to the following Examples.

### <Evaluation of Disulfide Bond Cleavage Activity of PDI Family Protein>

A disulfide bond cleavage test of an RBD of SARS-CoV-2 (B lineage) was performed using a gene recombinant of human PDI family proteins (PDI: SEQ ID NO: 4, ERp46: SEQ ID NO: 8, ERp57: SEQ ID NO: 12, ERp72: SEQ ID NO: 16, P5: SEQ ID NO: 20).

To a PBS buffer solution were added 0.5 µM of the gene recombinant of the PDI family protein and 2.5 mM of reduced glutathione, followed by standing at 37°C for 10 minutes. After 10 minutes, 2.5 µM of RBD of SARS-CoV-2 was added and a reaction was started at 37°C. The reaction solution at 0 minutes, 1 minute, 5 minutes, 10 minutes, 30 minutes, 60 minutes, and 180 minutes after the start of the reaction was reacted with 4 mM of maleimide-PEG 2k specifically added to a thiol group, and followed by performing analysis by 10% SDS-PAGE.

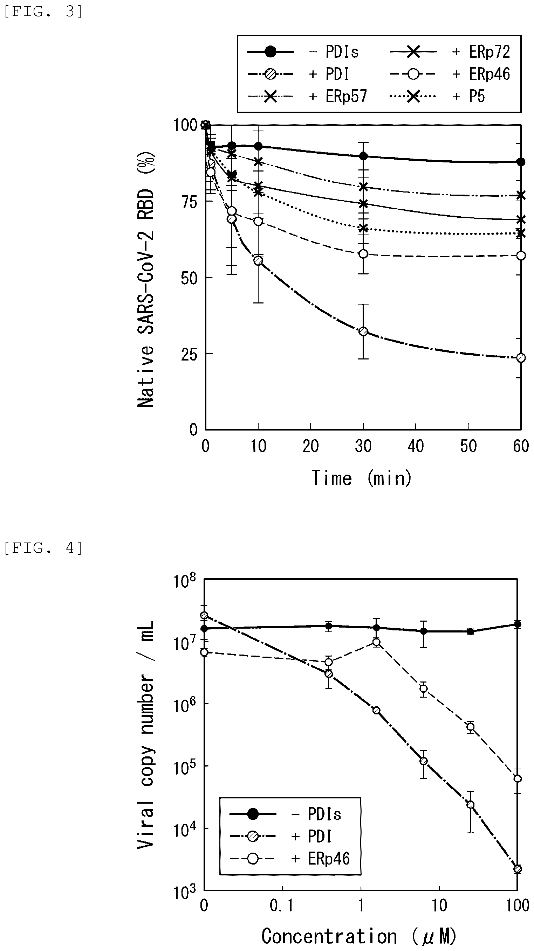

Results are shown in FIG. 3. FIG. 3 shows the disulfide bond cleavage activity as a remaining rate of a native RBD. In FIG. 3, "-PDIs" indicates that none of the PDI family proteins were added to the reaction solution, and each of "+PDI", "+ERp57", "+ERp72", "+ERp46", and "+P5" indicates that the described PDI family protein was added to the reaction solution. All of the tested PDI family proteins showed an activity to cleave the disulfide bond of the RBD. The order of the disulfide bond cleavage activity of the RBD from highest to lowest was PDI, ERp46, P5, ERp72, and ERp57. Among these, the disulfide bond cleavage activity of PDI was significantly high.

### <Evaluation of Antiviral Activity of PDI Family Protein>

The antiviral activity of the PDI family protein (PDI, ERp46) was evaluated using airway organoids. The airway organoids were prepared by a method previously reported (Emi Sano et al., Communications Biology volume 5, Article number: 516 (2022)). After 24 hours of seeding the airway organoids in a 96-well plate, a culture medium containing the PDI family protein was applied. an infection with SARS-CoV-2 (MOI = 0.1) was performed immediately thereafter. A cell culture supernatant was collected at 48 hours after infection, and a genome amount of SARS-CoV-2 contained therein was quantified. A method for quantifying the genome amount of SARS-CoV-2 contained in the cell culture supernatant was as follows.

The cell culture supernatant was mixed with an equal volume of a 2×RNA dissolution buffer (distilled water containing 0.4 U/µL of SUPERase InRNase Inhibitor (manufactured by Thermo Fisher Scientific), 2 v/v% of Triton X-100, 50 mM of KCl, 100 mM of Tris-HCl (pH 7.4), and 40 v/v% of glycerol), and the mixture was incubated at room temperature for 10 minutes. The mixture was diluted 10-fold with the distilled water. Viral RNA was quantified using a One Step TB Green PrimeScript PLUS RT-PCR kit (Perfect Real Time) (manufactured by Takara Bio Inc.) on a QuantStudio 1 Real-Time PCR system (manufactured by Thermo Fisher Scientific). A calibration curve was created using SARS-CoV-2 RNA (10⁵ copies/uL) purchased from Japan Gene Research Institute Co., Ltd. Sequences of primers used in RT-PCR were shown below.
Forward primer: AGCCTCTTCTCGTTCCTCATCAC (SEQ ID NO: 21)
Reverse primer: CCGCCATTGCCAGCCATTC (SEQ ID NO: 22)

Results are shown in FIG. 4. FIG. 4 shows an antiviral effect as the number of viral copies of SARS-CoV-2. In FIG. 4, "-PDIs" indicates that none of the PDI family proteins were added to the culture medium, and each of "+PDI" and "+ERp46" indicates that the described PDI family protein was added to the culture medium. Both PDI and ERp46 inhibited the SARS-CoV-2 infection in a concentrationdependent manner. It was confirmed from these results that PDI and ERp46 had the high antiviral activity against SARS-CoV-2. It is considered that the PDI family protein inhibits the binding of SARS-CoV-2 to ACE2 by cleaving the disulfide bond of the RBD, thereby inhibiting a host cell from being infected.

### <Evaluation of Cytotoxicity of PDI>

Whether PDI has cytotoxicity against human cells was evaluated. After 24 hours of seeding the airway organoids in a 96-well plate, a culture medium containing the PDI family protein was applied. After 48 hours of application of the PDI family protein, a cell viability was evaluated. The cell viability was measured using Cell Counting Kit-8 (DOJINDO LABORATORIES).

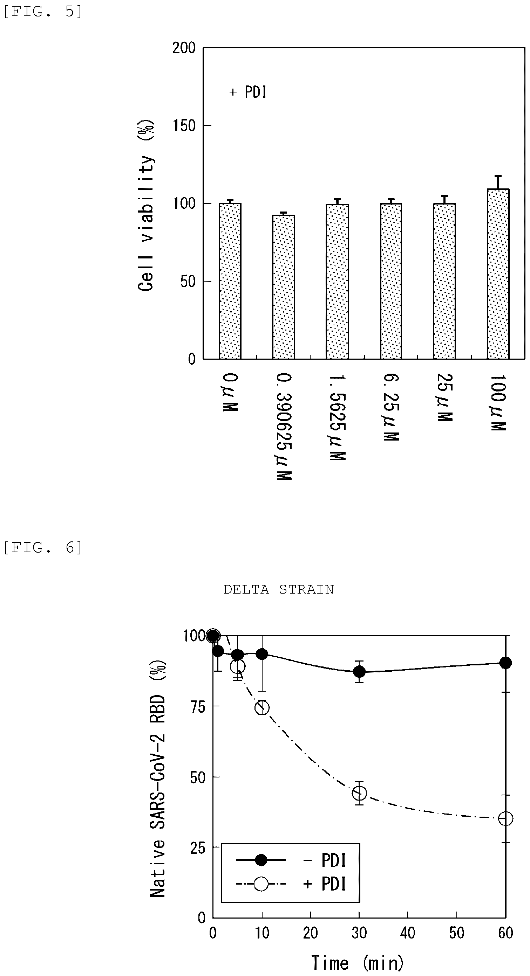

Results are shown in FIG. 5. FIG. 5 shows a cytotoxic activity of PDI as the cell viability. It was confirmed that PDI had no cytotoxicity against human cells. From these results, it is considered that PDI has a low risk of side effects.

### <Evaluation of Disulfide Bond Cleavage Activity Against SARS-CoV-2 Mutant Strains>

The disulfide bond cleavage activity of PDI was evaluated in the same manner as in <Evaluation of Disulfide Bond Cleavage Activity of PDI Family Protein> described above, except that RBDs of SARS-CoV-2 mutant strains (Delta strain, Omicron strain) were used instead of the RBD of SARS-CoV-2 (B lineage).

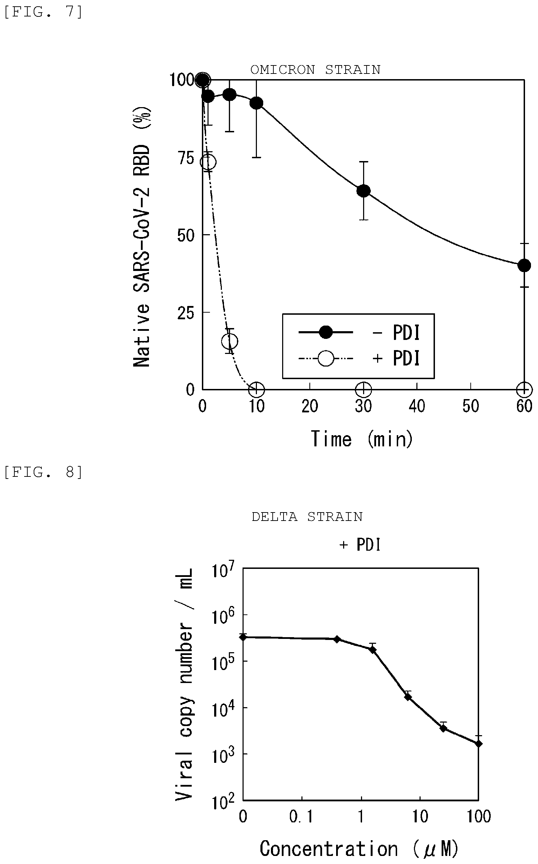

Results for the RBD of the Delta strain are shown in FIG. 6, and results for the RBD of the Omicron strain are shown in FIG. 7. FIGS. 6 and 7 show the disulfide bond cleavage activity as the remaining rate of the native RBD. In FIGS. 6 and 7, "-PDI" indicates that PDI was not added to the reaction solution, and "+PDI" indicates that PDI was added to the reaction solution. PDI showed an activity to cleave disulfide bonds in both the RBD of the Delta strain and the RBD of the Omicron strain. The disulfide bond cleavage activity was particularly high in the RBD of the Omicron strain.

### <Evaluation of Antiviral Activity of PDI Against SARS-CoV-2 Mutant Strains>

The antiviral activity of PDI against the SARS-CoV-2 mutant strains (Delta strain, Omicron strain) was evaluated. After 24 hours of seeding the airway organoids in the 96-well plate, a culture medium containing PDI was applied. An infection with the SARS-CoV-2 mutant strains (MOI = 0.1) was performed immediately thereafter. A cell culture supernatant was collected at 48 hours after infection, and a genome amount of SARS-CoV-2 mutant strains contained therein was quantified. All of the SARS-CoV-2 mutant strains used in this experiment were isolated from COVID-19 patients. Sequence information for a Delta strain B. 1.617.2 (GISAID accession number: EPI_ISL_9636792), an Omicron strain B.1.1.529, and BA1 (EPI_ISL_9638489) is registered in GISAID.

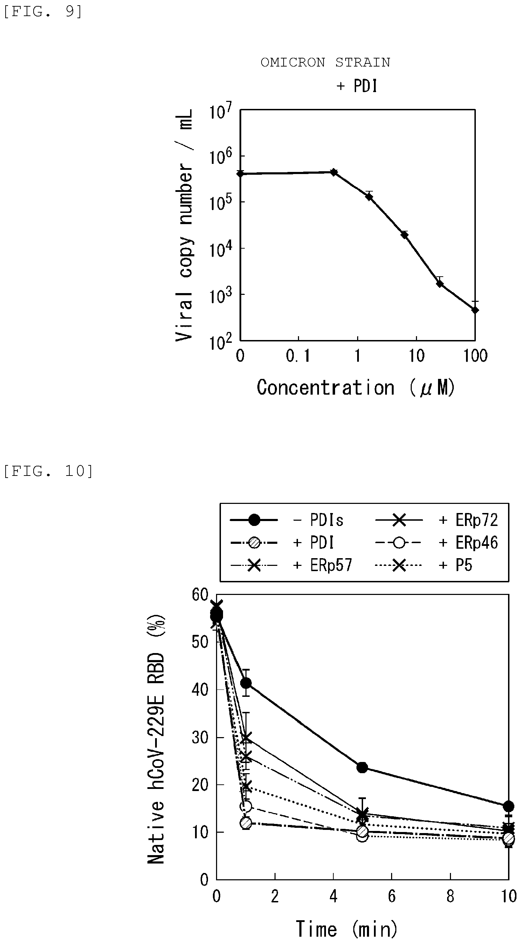

FIG. 8 shows results of the evaluation of the antiviral activity of PDI against the Delta strain. FIG. 9 shows results of the evaluation of the antiviral activity of PDI against the Omicron strain. PDI showed the high antiviral activity against both the Delta strain and the Omicron strain. The PDI family protein inhibits the binding of SARS-CoV-2 to ACE2 by cleaving the disulfide bond of the RBD and changing a three-dimensional structure of the RBD, and thus, it is considered that the PDI family protein also maintains a high infection inhibiting effect against the mutant strains.

### <Evaluation of Disulfide Bond Cleavage Activity Against RDB of hCoV-229E>

The disulfide bond cleavage activity of PDI was evaluated in the same manner as in <Evaluation of Disulfide Bond Cleavage Activity of PDI Family Protein> described above, except that an RBD of hCoV-229E was used instead of the RBD of SARS-CoV-2 (B lineage).

Results are shown in FIG. 10. FIG. 10 shows the disulfide bond cleavage activity as the remaining rate of the native RBD. In FIG. 10, "-PDIs" indicates that none of the PDI family proteins were added to the reaction solution, and each of "+PDI", "+ERp57", "+ERp72", "+ERp46", and "+P5" indicates that the described PDI family protein was added to the reaction solution. All of the tested PDI family proteins showed an activity to cleave the disulfide bond of the RBD of hCoV-229E. The order of the disulfide bond cleavage activity of the RBD of hCoV-229E from highest to lowest was PDI, ERp46, P5, ERp75, and ERp72. Among these, the disulfide bond cleavage activity of PDI was significantly high.

### Industrial Applicability

According to the invention, an antiviral agent that can maintain an antiviral activity against a mutant strain of a causative virus of a virus infection, and a pharmaceutical composition for treating or preventing a virus infection and containing the antiviral agent are provided. In addition, a virus infection severity risk evaluation kit and a virus infection severity risk evaluation method are provided.

The preferred Examples of the invention have been described above, but the invention is not limited to Examples. In a scope not departing from the gist of the invention, additions, omissions, replacements, and other changes of the configuration can be made. The invention is not limited to the above description, and is limited only by the appended claims.

## Claims

1. An antiviral agent comprising:
at least one selected from the group consisting of (a) a protein having a disulfide bond cleavage activity, and (b) a polynucleotide containing a nucleotide sequence encoding the protein.

2. The antiviral agent according to claim 1, wherein
the protein is a protein disulfide isomerase family protein.

3. The antiviral agent according to claim 2, wherein
the protein disulfide isomerase family protein is at least one selected from the group consisting of PDI, ERp46, ERp57, ERp72, and P5.

4. The antiviral agent according to claim 3, wherein
the protein disulfide isomerase family protein is at least one selected from the group consisting of PDI and ERp46.

5. The antiviral agent according to claim 1, which is to be applied to a virus having a disulfide bond in at least one protein selected from the group consisting of a spike protein, an envelope protein, and a capsid protein.

6. A pharmaceutical composition for treating or preventing a virus infection, the pharmaceutical composition comprising:
the antiviral agent according to any one of claims 1 to 5; and
a pharmaceutically acceptable carrier.

7. A virus infection severity risk evaluation kit comprising:
a specific binding substance for a protein having a disulfide bond cleavage activity.

8. A virus infection severity risk evaluation method comprising:
a step of measuring a concentration of a protein having a disulfide bond cleavage activity in a biological sample from a test subject.

9. A determination method comprising:
a step of measuring a concentration of a protein having a disulfide bond cleavage activity in a biological sample from a test subject;
a step of evaluating a severity risk of a virus infection in the test subject based on the concentration of the protein having a disulfide bond cleavage activity; and
a step of determining whether the test subject determined to have a high severity risk is a test subject to be administered with an antiviral agent.
